Europäisches Patentamt

European Patent Office

Office européen des brevets

(11) Publication number: **0 075 136**
**B1**

(12) EUROPEAN ·PATENT SPECIFICATION

(45) Date of publication of patent specification: **12.03.86**

(51) Int. Cl.⁴: **C 07 C 41/34, C 07 C 43/04**

(21) Application number: **82107906.8**

(22) Date of filing: **27.08.82**

(54) A process for recovering methyl-tert-butyl ether from a reaction effluent.

(30) Priority: **28.08.81 US 297456**

(43) Date of publication of application:
**30.03.83 Bulletin 83/13**

(45) Publication of the grant of the patent:
**12.03.86 Bulletin 86/11**

(84) Designated Contracting States:
**AT BE CH DE FR GB IT LI LU NL SE**

(56) References cited:
**FR-A-2 449 666
GB-A-2 047 706
GB-A-2 049 693
US-A-4 144 138**

(73) Proprietor: **PHILLIPS PETROLEUM COMPANY
5th and Keeler
Bartlesville Oklahoma 74004 (US)**

(72) Inventor: **Van Pool, Joe
1418 Prairie Heights Drive
Bartlesville Oklahoma 74003 (US)**
Inventor: **Hann, Paul Douglas
1313 S.E. Woodridge Drive
Bartlesville Oklahoma 74003 (US)**

(74) Representative: **Dost, Wolfgang, Dr.rer.nat.,
Dipl.-Chem. et al
Patent- und Rechtsanwälte Bardehle-
Pagenberg-Dost-Altenburg & Partner Postfach
86 06 20
D-8000 München 86 (DE)**

Courier Press, Leamington Spa, England.

## Description

This invention relates to a process for the recovery of methyl-tert-butyl ether (MTBE). In accordance with a further aspect, this invention relates to an ether recovery system when driers are not used on the hydrocarbon feed to MTBE manufacture. In a further aspect, this invention relates to an ether recovery system when driers are used on the hydrocarbon feed to MTBE manufacture. In still another aspect, this invention relates to a combination process for the recovery of ethers and unreacted materials, the hydrocarbon portion of which can be passed to alkylation.

Methyltertiarylbutyl ether (MTBE) is well-known as a high octane blending component for motor fuels. The well-known reaction of methanol (MeOH) and isobutylene, using an appropriate catalyst, such as Amberlyst 15®, has been practiced to produce MTBE. Reference is had to U.S. patents 4,071,567; 3,979,461; 3,135,807; 3,846,088; among many others.

HF catalytic alkylation of isobutane with olefins, such as propylene and/or butylenes, is also a well-known process for producing high octane motor fuel. In HF alkylation it is also known that too much water in HF catalyst can adversely affect the alkylation operation, including presenting problems of equipment corrosion. In addition, methanol is not wanted to be present in HF alkylation since methanol uses isobutane in its reaction therewith to produce undesired low octane, high volatility five carbon-atom hydrocarbons and water, the water undesiredly diluting the HF catalyst.

Since there is some unreacted methanol and there is some unreacted isobutylene in the MTBE reactor effluent, it is desired to recover these components and to recycle the methanol, preferably, back to the MTBE reaction; and to recover the unreacted isobutylene (along with isobutane and straight chain butylenes, which hydrocarbons are present in the feed to MTBE and act as desired diluents in the MTBE reaction) and charge this recovered isobutylene, freed from methanol, to the HF alkylation.

It is desired that no methanol be yielded, and thereby lost, in the MTBE product. It is desired that substantially no water and no methanol be charged to the HF alkylation.

FR—A—2 449 666 discloses a process for the isolation of MTBE from a reaction effluent containing methanol, MTBE and C₄ hydrocarbons. The reaction effluent is fractionated into a bottom fraction comprising MTBE and an overhead stream comprising $C_4$ hydrocarbon and methanol. The total overhead stream is washed with water and subsequently phase separated into a hydrocarbon phase and a liquid phase comprising methanol and water. The hydrocarbon phase contains up to about 10 percent of the total methanol content of the overhead stream, which is too high to subject this hydrocarbon phase to subsequent alkylation. Accordingly, this reference neither discloses nor suggests to subject the hydrocarbon phase obtained after washing with water to an alkylation step.

GB—A—2 049 693 discloses a process for recovering MTBE wherein $C_5$ and/or $C_6$ hydrocarbons are used as azeotropic agents for carrying away the methanol. The reaction effluent is separated in a first fractionation step to a top product comprising $C_4$ hydrocarbons and a bottom product comprising methanol, MTBE, and the $C_5$ and/or $C_6$ hydrocarbons. In order to recover a MTBE product free of methanol a second fractionation step of the bottom product is necessary. The top product of the first fractionation step comprising $C_4$ hydrocarbons is discharged, after optionally having been washed with water. This reference does not suggest to subject the hydrocarbon phase to a further processing.

The process of the invention overcomes the disadvantages of the afore-mentioned prior art, i.e. it yields a hydrocarbon phase substantially free of methanol and a MTBE product free of methanol in only one fractionation step.

Accordingly the invention presents a system: to produce MTBE from methanol and isobutylene with the MTBE being recovered free of methanol and water; to recover methanol and unreacted hydrocarbons; to separate a water and methanol phase from the unreacted hydrocarbons; to water-wash the unreacted hydrocarbons to remove the last traces of methanol therefrom; and to dry these water-washed hydrocarbons prior to charging the methanol-free, water-free hydrocarbons to HF alkylation; to fractionate the water-methanol phase, above-referred-to, to recover substantially pure methanol which can be recycled to MTBE manufacture; and to use the water separated from the methanol as at least part of the water used in the water-wash step, above-referred-to.

Accordingly, an object of this invention is to provide a process for preparing and recovering methyl-tert-butyl ether.

Another object of this invention is to recover unreacted materials for recycle following the ether reaction.

A further object of this invention is to recover unreacted hydrocarbons in an ether effluent for further use in an alkylation process.

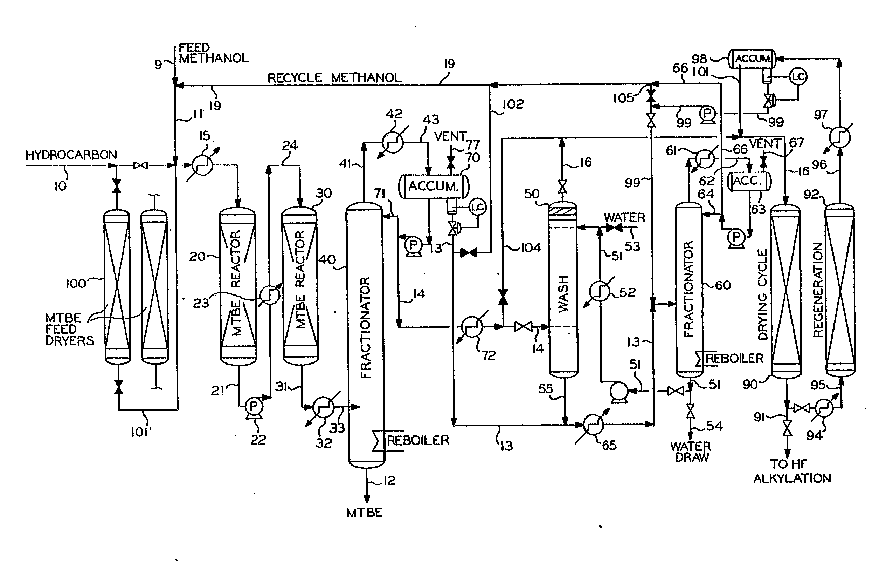

Other objects, aspects, as well as the several advantages of the invention will be apparent to those skilled in the art upon reading the specification, the drawing and the appended claims.

In accordance with the invention a process for recovering MTBE from a reaction effluent containing MTBE, methanol, unreacted hydrocarbons comprising isobutylene and other $C_4$ hydrocarbons, and water is provided, wherein said effluent is fractionated in a first fractionation step to obtain an overhead stream comprising unreacted hydrocarbons, methanol and water and a bottoms fraction comprising MTBE substantially free of methanol, said overhead is condensed and subjected to a further treatment comprising the steps of washing with water and phase separating into an upper hydrocarbon phase and a lower liquid phase comprising methanol and water and said lower liquid phase is passed together with a water stream recovered from the water washing step to a second fractionation step to separately recover a methanol stream and a water stream which process is characterized in that said water washing step is carried out after phase separating the condensed overhead to recover a hydrocarbon stream substantially free of methanol and water, and a water stream containing methanol.

The lower liquid phase comprising methanol and water which is obtained as a heavier aqeuous phase in an MTBE fractionator overhead accumulator cannot be recycled to the first fractionation step, i.e. the MTBE fractionator, because the bottom product MTBE will then be contaminated with methanol and water. This heavier aqueous phase is separately processed in a second fractionation step, i.e. a methanol fractionator, to recover a methanol stream and a water stream; in particular this methanol stream recycled to the reaction to form MTBE and the water stream is used as at least a portion of the water used for the water washing step.

According to a specific embodiment the hydrocarbon stream obtained after the water washing step is passed through a drying zone and then to an alkylation zone for contact with an olefin and an alkylation catalyst to form an alkylate; in particular at least a portion of the effluent from the drying zone is used to regenerate another drier on a regeneration cycle, and the effluent from the drier on the regeneration cycle is condensed and separated into a hydrocarbon phase which is passed to a drier on a drying cycle and a water phase containing methanol which is passed as part of the feed for said fractionation step.

If the lower liquid phase obtained after phase-separating the condensed overhead into an upper hydrocarbon phase and a lower liquid phase comprises substantially methanol, as in a further embodiment, said lower liquid phase comprising substantially methanol is recycled to MTBE manufacture to form said reaction effluent, and said hydrocarbon phase is passed to an on-stream drier preceding an alkylation to remove the last traces of methanol.

The methanol liquid phase cannot be recycled to the MTBE fractionator because it will allow methanol to be in the bottom MTBE product, contaminating the MTBE, and causing a recovery problem or loss of methanol.

The hydrocarbon phase obtained after phase separation, containing methanol, passes by the water washing step and is charged to feed driers for a HF alkylation, wherein the methanol is absorbed, yielding methanol-free hydrocarbons, which are charged to HF alkylation; in particular a portion of the hydrocarbon product from the on-stream drier is heated and vaporized and used to regenerate the drier on a regeneration cycle, i.e. used to desorb methanol from that drier. The vaporous effluent from this regeneration is condensed and phase-separated into a hydrocarbon phase which is recycled to the on-stream drier and a methanol phase which is recycled to MTBE manufacture. Both the water washing step and the methanol fractionator are bypassed.

According to a further specific embodiment the hydrocarbon feed is passed through driers to remove water prior to being passed to MTBE manufacture for forming the reaction effluent.

When MTBE feed driers are used and when insufficient methanol is present in the MTBE reactor effluent to form a separate liquid phase in the MTBE fractionator overhead accumulator, the yield from the accumulator is charged to the HF alkylation feed driers, as described hereinabove, by-passing the water-wash column. Also, the methanol fractionation is not needed for this operation.

It is pointed out that when driers are not used on the feed to MTBE and when the methanol-water phase occurs in the MTBE fractionator overhead accumulator, that the hydrocarbon phase has both water and methanol therein, and after water washing this stream, the washed hydrocarbon is passed to the HF alkylation feed driers. The drier on regeneration can use heated, vaporized dried hydrocarbon for regeneration, and the vapor effluent, hydrocarbon and water, with a trace of methanol, is passed via condensing to a phase separator. The hydrocarbon phase from this accumulator or separator is returned to that drier on the drying cycle, and the aqueous phase is charged to the methanol fractionator, recovering methanol for recycle to the MTBE manufacture.

Given the foregoing description, one skilled in the art having studied the same can determine by mere routine testing the design and conditions of operation required to carry out the invention. However, to more fully describe the invention and to set forth a now best mode contemplated for it in its application to the recovery of the effluent from an ether operation reference is had to the drawing.

Referring now to the drawing, and operating without driers on the hydrocarbon feed to MTBE manufacture, hydrocarbon feed 10, comprising reactant isobutylene and non-reactant, or diluent, isobutane, straight chain butylenes, and normal butane from the field, as from a catalytic cracking operation, is admixed with methanol (feed 9 and recycle 19) added via a conduit 11, and the mass is passed via heater 15 to MTBE reactor 20 containing Amberlyst 15 catalyst or other suitable catalyst. Effluent 21 from reactor 20 is pumped with a pump 22 via indirect heater 23 and conduit 24 into the second reactor 30, also containing Amberlyst 15 catalyst.

Effluent 31 from reactor 30 is indirectly heated at an indirect heater 32 and passed via conduit 33 to reboiled and refluxed MTBE fractionator 40. MTBE product, substantially free of methanol, is recovered in a conduit 12 for use, for example, as a high octane blending component in motor fuel.

The overhead vapor stream 41 from fractionator 40 is indirectly cooled and substantially all condensed in 42 and passed via a conduit 43 to accumulator 70. Inerts can be removed from accumulator 70 via conduit 77.

In accumulator 70 two liquid phases are formed. The lower liquid phase comprises water and unreacted methanol. The upper liquid phase comprises unreacted hydrocarbons and contains some water and unreacted methanol. The lower liquid phase is passed via conduit 13 and indirect heater 65 to methanol fractionator 60 as part of the feed thereto. The upper hydrocarbon phase from accumulator 70 in part refluxes fractionator 40 via conduit 71, and the yield portion of hydrocarbon phase is passed via conduit 14 and indirect cooler 72 to water wash column 50 wherein the liquid hydrocarbon phase containing some methanol is washed with liquid water added to the wash column 50 via conduit 51.

Water, containing substantially no methanol, from methanol fractionator 60 is passed via conduit 51 and is pumped via indirect cooler 52 to water wash column 50. Makeup water can be added at conduit 53. Yield water can be removed at conduit 54. Water containing methanol is passed from column 50 via conduit 55 into methanol fractionator 60 along with the material in conduit 13.

Hydrocarbon, substantially freed of methanol but containing solution water (which will also contain some methanol), is recovered at conduit 16 and passed to the HF alkylation feed drier 90 which is on the drying cycle. The dried, methanol-free hydrocarbon is charged via conduit 91 to an HF alkylation, not shown, to effect alkylation of isobutane with propylene and/or butylenes, some of which olefins are present in the stream in conduit 91. Outside isobutane and propylene and/or butylenes can also be charged to the HF alkylation.

Drier 92 is on the regeneration cycle, preferably using as regeneration fluid a portion of the dried hydrocarbon from drier 90 passed via indirect heater-vaporizer 94 and conduit 95 through drier 92 for regeneration. Vaporous effluent 96 from drier 92 is condensed in exchanger 97 and passed to liquid phase separator 98. The lower aqueous phase (methanol and water) is (pumped) passed via conduit 99 to the methanol fractionator 60 for recovery of methanol for recycle to MTBE manufacture. The hydrocarbon phase is recycled via conduit 101 and conduit 16 to drier 90 on the hydrocarbon drying cycle.

The overhead vaporous methanol from methanol fractionator 60 is condensed in indirect heat exchanger condenser 61 and is passed via conduit 62 to overhead accumulator 63. Liquid methanol refluxes fractionator 60 via a conduit 64 and the yield methanol portion is passed via conduits 66 and 19 back to MTBE manufacture.

Inerts can be vented from accumulator 63 via conduit 67.

When MTBE hydrocarbon feed drier 100 is used, and the dried hydrocarbon is charged via a conduit 101' to MTBE manufacture, and when sufficient excess methanol is in the reactor effluent, the lower liquid phase formed in accumulator 70 is substantially only methanol. This methanol can be passed via conduits 102 and 19 for recycle to the MTBE manufacture. It is not recycled to fractionator 40 since this would cause methanol to go out with MTBE product. The upper hydrocarbon phase containing some methanol to be yielded is passed via conduit 14, via conduit 104, and via conduit 16 to the on-stream drier 90 to remove the last traces of methanol from the hydrocarbon. On regeneration of the spent drier 92, the dried hydrocarbon from the on-stream drier 90 is passed via indirect heater-vaporizer 94 through drier 92 for regeneration thereof. Vaporous effluent from drier 92 is condensed in indirect exchanger 97 and is passed to liquid phase separator 98. The lower methanol phase is recycled via conduits 99, 105, and 19 to MTBE manufacture. The upper hydrocarbon phase is returned via conduits 101 and 16 to drier 90, as illustrated in the drawing.

When there is insufficient methanol in the feed passed via conduit 33 to the MTBE fractionator, there is no separate methanol phase formed in the overhead accumulator, and, of course, there need be no flow from the leg of accumulator 70 via conduit 13. The hydrocarbon phase containing methanol in solution (this is the operation using drier 100), is used as reflux via conduit 71, in part, and yielded via conduits 14, 104, and 16 to drier 90 for removal of the soluble methanol therefrom.

It is pointed out that fractionator 40 is operated so that substantially no methanol exits the bottom thereof along with product MTBE, but that the methanol azeotropes (overhead) with the light hydrocarbons. Pressures and temperaturs other than the specific temperatures and pressures tabulated can be selected by those skilled in this fractionation art.

This fractionator 40 is refluxed with separated liquid hydrocarbon, and not ever with the water-methanol phase, which latter stream would cause methanol and water to exit in part in the MTBE, and then this MTBE stream would have to be treated for water and methanol removal and recovery of methanol.

The driers can use conventional molecular seive 5A, 13X, or the like. Activated alumina can also be used as the desiccant, as is known in the art. Conventional conditions for drying and regeneration are employed in the driers.

Not all of the valves, pumps, heat exchangers, and control systems are shown on the drawing in order to simplify the drawing. Pertinent controls, valves, exchangers, etc. are illustrated, however.

**0 075 136**

Calculated Example

Operating conditions:

Reactors:

| (20) | Temperature at inlet, °F., (°C) | 130 (54) |
|---|---|---|
| | Temperature at Outlet, °F., (°C) | 155 (68) |
| | Pressure at Inlet, psia., (MPa) | 180 (1.24) |
| | Catalyst, | Amberlyst 15 |
| | LHSV, v/v/hr[a] | 5 |
| | Isobutylene/Methanol Feed Mol Ratio, | 0.72:1.0 |

| (30) | Temperature at inlet, °F., (°C) | 110 (43) |
|---|---|---|
| | Temperature at Outlet, °F., (°C) | 115 (46) |
| | Pressure at Inlet, psia., (MPa) | 180 (1.24) |
| | Catalyst, | Amberlyst 15 |
| | LHSV, v/v/hr | 5 |

Fractionator (40):

Temperatures:
Top, °F., (°C) — 122 (50)
Bottom, °F., (°C) — 236 (113)

Pressures:
Top, psia., (MPa) — 80 (0.55)
Bottom psia., (MPa) — 85 (0.58)

Water wash vessel (50):
Temperature, °F, (°C) — 100 (38)
Pressure, psia., (MPa) — 120 (0.83)

Methanol fractionator (60):

Temperatures:
Top, °F., (°C) — 169 (76)
Bottom, °F., (°C) — 240 (115)

Pressures:
Top, psia., (MPa) — 23 (0.16)
Bottom, psia., (MPa) — 25 (0.17)

Accumulator (70)
Temperature, °F., (°C) — 109 (42)
Pressure, psia., (MPa) — 75 (0.52)

Accumulator (63)
Temperature, °F., — 164 (73)
Pressure, psia., — 20 (0.14)

| Flow rates: | | | Pounds/Hour | kg/h |
|---|---|---|---|---|
| (10) | Hydrocarbon Feed, | | 100,000 | 45300 |
| | Wt.% Isobutylene, | 25.9 | | |
| | Wt.% Water, | 0.05 | | |
| (11) | Fresh (Feed) and Recycle Methanol, | | 12,309.5 | 5576 |
| | Wt.% water, | 0.26 | | |
| (12) | MTBE Product, | | 46,240 | 20947 |
| | Wt.% MTBE | 87.9 | | |
| | Wt.% Hydrocarbon | 12.1 | | |
| | Wt.% Water, | 0 | | |
| | Wt.% Methanol, | 0 | | |

5

Calculated Example (Cont.)

| | | | | | |
|---|---|---|---|---|---|
| (13) | Methanol-Water, | | | 325.2 | 147.3 |
| | Wt.% Water, | 16.8 | | | |
| | Wt.% Methanol, | 83.2 | | | |
| (14) | Hydrocarbon to Water Wash, | | | 65,744.3 | 29782 |
| | Wt.% Water, | 0.083 | | | |
| | Wt.% Methanol, | 0.274 | | | |
| (16) | Hydrocarbon From Water Wash, | | | 65,581.3 | 29708 |
| | Wt.% Water, | 0.003 | | | |
| | Wt.% Methanol, | 0.107 | | | |
| (55) | Aqueous Stream | | | 10,231.9 | 4635 |
| | Wt.% Methanol, | 1.89 | | | |
| (66) | Recovered Methanol, | | | 458 | 207.5 |
| | Wt.% Water, | 1.48 | | | |
| (51) | Aqueous Liquid | | | 10,072.8 | 4563 |
| | Wt.% Methanol, | 0.13 | | | |
| (54) | Water Removal, | | | 72.8 | 33 |
| | Wt.% Methanol, | 0.13 | | | |

[a] LHSV is volumes of liquid per volume of catalyst per hour

## Claims

1. A process for recovering methyl-tertiarybutyl ether (MTBE) from a reaction effluent containing MTBE, methanol, unreacted hydrocarbons comprising isobutylene and other $C_4$ hydrocarbons, and water, wherein said effluent is fractionated in a first fractionation step to obtain an overhead stream comprising unreacted hydrocarbons, methanol and water and a bottoms fraction comprising MTBE substantially free of methanol, said overhead is condensed and subjected to a further treatment comprising the steps of washing with water and phase-separating into an upper hydrocarbon phase and a lower liquid phase comprising methanol and water, and said lower liquid phase is passed together with a water stream recovered from the water washing step to a second fractionation step to separately recover a methanol stream and a water stream characterized in that said water washing step to recover a hydrocarbon stream substantially free of methanol and water, and a water stream containing methanol, is carried out after phase-separating said condensed overhead into said upper hydrocarbon phase and said lower liquid phase.

2. A process according to claim 1 characterized in that said hydrocarbon stream is passed through a drying zone and then to an alkylation zone for contact with an olefin and an alkylation catalyst to form an alkylate.

3. A process according to claim 2 characterized in that at least a portion of the effluent from the drying zone is used to regenerate another drier on a regeneration cycle, and the effluent from the drier on regeneration cycle is condensed and separated into a hydrocarbon phase which is passed to a drier on a drying cycle and a water phase containing methanol which is passed as part of the feed for said second fractionation step.

4. A process according to one of claims 1 to 3 characterized in that said methanol stream recovered after said second fractionation step is recycled to the reaction to form MTBE and the water stream obtained after said second fractionation step is used as at least a portion of the water used for the water-washing step.

5. A process for recovering MTBE from a reaction effluent containing MTBE, unreacted hydrocarbons comprising isobutylene and other $C_4$ hydrocarbons, methanol, and minor amounts of water comprising fractionating said effluent to obtain an overhead fraction comprising unreacted hydrocarbons, methanol, and water, if any, and a bottoms fraction comprising MTBE substantially free of methanol, condensing said overhead and phase separating said condensed overhead into an upper hydrocarbon phase and a lower liquid phase comprising substantially methanol, characterized in that said upper hydrocarbon phase is passed to an on-stream drier preceding an alkylation to remove the last traces of methanol; and said lower liquid phase comprising substantially methanol is recycled to MTBE manufacture to form said reaction effluent.

6. A process according to claim 5 characterized in that at least a part of the effluent from said on-stream drier is used to regenerate a drier on a regeneration cycle, and the effluent from the drier on regeneration is condensed and separated into a hydrocarbon phase which is passed to a drier on a drying cycle and methanol which is recycled to MTBE manufacture.

7. A process according to claim 5 or 6 characterized in that the hydrocarbon feed is passed through driers to remove water prior to being passed to MTBE manufacture for forming said reaction effluent.

8. A process according to one of claims 5 to 7 characterized in that sufficient excess methanol is used in the MTBE manufacture and forming said reaction effluent that essentially only methanol is formed as lower liquid phase.

**Patentansprüche**

1. Verfahren zur Gewinnung von Methyl-tert.-butyl-ether (MTBE) aus einem Reaktionsabfluß, der MTBE, Methanol, unumgesetzte Kohlenwasserstoffe, die Isobutylen und andere $C_4$-Kohlenwasserstoffe umfassen, und Wasser enthält, wobei der genannte Abfluß in einer ersten Fraktionierungsstufe fraktioniert wird, um einen Überkopfstrom, der unumgesetzte Kohlenwasserstoffe, Methanol und Wasser enthält, und eine Bodenfraktion, die MTBE im wesentlichen frei von Methanol enthält, zu erhalten, das genannte Überkopfprodukt kondensiert wird und einer weiteren Behandlung unterworfen wird, die die Schritte Wasserwäsche und Phasentrennung in eine obere Kohlenwasserstoffphase und eine untere flüssige Phase enthaltend Methanol und Wasser umfaßt, und die genannte untere flüssige Phase mit einem Wasserstrom, der vom Schritt Wasserwäsche gewonnen wird, zu einer zweiten Fraktionierungsstufe geleitet wird, um einen Methanol- und einen Wasserstrom getrennt zu gewinnen, dadurch gekennzeichnet, daß der genannte Schritt Wasserwäsche zur Gewinnung eines im wesentlichen von Methanol und Wasser freien Kohlenwasserstoffstromes und eines Methanol enthaltenden Wasserstromes nach der Phasentrennung des kondensierten Überkopf-produktes in die genannte obere Kohlenwasserstoffphase und die genannte untere flüssige Phase ausgeführt wird.

2. Verfahren nach Anspruch 1, dadurch gekennzeichnet, daß der genannte Kohlenwasserstoffstrom durch eine Trocknungszone geleitet wird und dann zum In-Berührung-Bringen mit einem Olefin und einem Alkylierungskatalysator in eine Alkylierungszone zwecks Bildung eines Alkylats.

3. Verfahren nach Anspruch 2, dadurch gekennzeichnet, daß zumindest ein Teil dès Abflusses aus der Trocknungszone zur Regenerierung eines anderen Trockners in einem Regenerierungskreis verwendet wird und der Abfluß aus dem Trockner im Regenerierungskreis kondensiert und in eine Kohlenwasserstoffphase, die zu einem Trockner in einem Trocknungskreis geleitet wird, und in eine Methanol enthaltende Wasserphase, die als Teil der Zufuhr für die genannte zweite Fraktionierungsstufe verwendet wird, getrennt wird.

4. Verfahren nach einem der Ansprüche 1 bis 3, dadurch gekennzeichnet, daß der genannte Methanolstrom, der nach der genannten zweiten Fraktionierungsstufe gewonnen wird, zur Reaktion zur Bildung von MTBE zurückgeleitet wird und der Wasserstrom, der nach der genannten zweiten Fraktionierungsstufe gewonnen wird, zumindest als Teil des Wassers für den Schritt der Wasserwäsche verwendet wird.

5. Verfahren zur Gewinnung von MTBE aus einem Reaktionsabfluß, der MTBE, unumgesetzte Kohlenwasserstoffe, die Isobutylen und andere $C_4$ Kohlenwasserstoffe umfassen, Methanol und geringe Mengen Wasser enthält, bei welchem der genannte Abfluß fraktioniert wird, um eine Überkopffraktion, die unumgesetzte Kohlenwasserstoffe, Methanol und gegebenenfalls Wasser enthält, und eine Bodenfraktion, die MTBE im wesentlichen frei von Methanol enthält, zu erhalten, das genannte Überkopfprodukt kondensiert und das genannte kondensierte Überkopfprodukt in eine obere Kohlenwasserstoffphase und eine untere flüssige Phase, die im wesentlichen Methanol umfaßt, getrennt wird, dadurch gekennzeichnet, daß die genannte obere Kohlenwasserstoffphase vorausgehend einer Alkylierung zu einem Durchflußtrockner geleitet wird, um die letzten Methanolspuren zu entfernen; und die genannte untere flüssige Phase, die im wesentlichen Methanol enthält, zur MTBE-Herstellung zurückgeleitet wird, um den genannten Reaktionsabfluß zu bilden.

6. Verfahren nach Anspruch 5, dadurch gekennzeichnet, daß zumindest ein Teil des Abflusses des genannten Durchflußtrockners zur Regenerierung eines Trockners in einem Regenerierungskreis verwendet wird, und der Abfluß des Trockners, der regeneriert wird, kondensiert und in eine Kohlenwasserstoffphase, die zu einem Trockner in einem Trocknungskreise geleitet wird, und Methanol, das zur MTBE-Herstellung zurückgeleitet wird, getrennt wird.

7. Verfahren nach Anspruch 5 oder 6, dadurch gekennzeichnet, daß die Kohlenwasserstoffzufuhr durch Trockner geleitet wird, um Wasser zu entfernen, bevor sie zur Bildung des Reaktionsabflusses zur MTBE-Herstellung geleitet wird.

8. Verfahren nach einem der Ansprüche 5 bis 7, dadurch gekennzeichnet, daß ausreichend überschüssiges Methanol in der MTBE-Herstellung und der Bildung des genannten Reaktionsabflusses verwendet wird, daß im wesentlichen nur Methanol als untere flüssige Phase gebildet wird.

**Revendications**

1. Procédé de récupération de l'éther méthyl-tert-butylique (MTBE) à partir d'un effluent de réaction contenant du MTBE, du méthanol, des hydrocarbures n'ayant pas réagi, comprenant de l'isobutylène et d'autres hydrocarbures en $C_4$, et de l'eau, dans lequel cet effluent est fractionné dans un premier stade de fractionnement pour obtenir un courant de têtes comprenant des hydrocarbures n'ayant pas réagi, du méthanol et de l'eau, et une fraction de queues comprenant du MTBE pratiquement exempt de méthanol, ces têtes étant condensées et soumises à un traitement ultérieur comprenant les stades de lavage à l'eau et de séparation de phases en une phase hydrocarbonée supérieure et une phase liquide inférieure comprenant du méthanol et de l'eau, et cette phase liquide inférieure est envoyée en même temps qu'un courant d'eau recueillie à partir du stade de lavage à l'eau dans un second stade de fractionnement pour recueillir séparément un courant de méthanol et un courant d'eau, caractérisé en ce que ce stade de lavage à l'eau pour recueillir un courant d'hydrocarbure pratiquement exempt de méthanol et d'eau, et un courant d'eau contenant du méthanol, est effectué après avoir soumis à une séparation de phases ces têtes condensées en cette phase hydrocarbonée supérieure et cette phase liquide inférieure.

2. Procédé selon la revendication 1, caractérisé en ce que ce courant d'hydrocarbure est envoyé à travers une zone de séchage puis dans une zone d'alkylation pour venir en contact avec une oléfine et un catalyseur d'alkylation pour former un alcoylat.

3. Procédé selon la revendication 2, caractérisé en ce qu'au moins une partie de l'effluent provenant de la zone de séchage est utilisé pour régénérer un autre séchoir en cycle de régénération, et en ce que l'effluent provenant du séchoir en cycle de régénération est condensé et séparé en une phase hydrocarbonée qui est envoyée dans un séchoir en cycle de séchage et une phase aqueuse contenant du méthanol qui est envoyée en tant que partie de l'alimentation pour ce second stade de fractionnement.

4. Procédé selon l'une des revendications 1 à 3, caractérisé en ce que ce courant de méthanol recueillie après ce second stade de fractionnement est recyclé dans la fraction pour former du MTBE et en ce que le courant d'eau obtenu après ce second stade de fractionnement est utilisé comme au moins une partie de l'eau utilisée pour le stade de lavage à l'eau.

5. Procédé pour recueillir du MTBE à partir d'un effluent de réaction contenant du MTBE, des hydrocarbures n'ayant pas réagi comprenant de l'isobutylène et d'autres hydrocarbures en $C_4$, du méthanol et des quantités mineures d'eau, comprenant le fractionnement de cet effluent pour obtenir une fraction de têtes comprenant des hydrocarbures n'ayant pas réagi, du méthanol et de l'eau, s'il y en a, et une fraction de queues comprenant du MTBE pratiquement exempt de méthanol, la condensation de ces têtes et la séparation de phases de ces têtes condensées en une phase hydrocarbonée supérieure et une phase liquide inférieure comprenant essentiellement du méthanol, caractérisé en ce que cette phase hydrocarbonée supérieure est envoyée à un séchoir en marche précédant une alkylation pour éliminer les dernières traces de méthanol; et en ce que cette phase liquide inférieure comprenant essentiellement du méthanol est recyclée à la fabrication du MTBE pour former cet effluent de réaction.

6. Procédé selon la revendication 5, caractérisé en ce qu'au moins une partie de l'effluent provenant de ce séchoir en marche est utilisée pour régénérer un séchoir en cycle de régénération, et en ce que l'effluent provenant du séchoir en régénération est condensé et séparé en une phase hydrocarbonée qui est envoyée dans le séchoir en cycle de séchage et du méthanol qui est recyclé dans la fabrication du MTBE.

7. Procédé selon les revendications 5 ou 6, caractérisé en ce que l'alimentation en hydrocarbure est envoyée à travers des séchoirs pour éliminer de l'eau avant d'être envoyé à la fabrication du MTBE pour former cet effluent de réaction.

8. Procédé selon l'une quelconque des revendications 5 à 7, caractérisé en ce que suffisamment de méthanol en excès est utilisé dans la fabrication du MTBE et la formation de cet effluent de la réaction pour qu'il ne se forme pratiquement que du méthanol comme phase liquide inférieure.

0 075 136

FEED METHANOL

RECYCLE METHANOL

HYDROCARBON

MTBE FEED DRYERS

MTBE REACTOR

MTBE REACTOR

FRACTIONATOR

REBOILER

MTBE

ACCUM.

VENT

WASH

WATER

FRACTIONATOR

REBOILER

WATER DRAW

ACC.

VENT

ACCUM

DRYING CYCLE

REGENERATION

REBOILER

TO HF ALKYLATION

1